# EUROPEAN PATENT APPLICATION

(11) **EP 0 856 580 A1**
(43) Date of publication of application: **05.08.1998**
(21) Application number: 96901484.4
(22) Date of filing: 25.01.1996
(51) Int. Cl.: C12N 15/12, C12N 15/67, C12P 21/02, C12Q 1/70, C07K 14/47, C07K 14/435, C12N 1/21

(54) **RECOMBINANT CONGLUTININ AND PROCESS FOR PRODUCING THE SAME**

(30) Priority: 17.08.1995 JP 209698/95; 02.10.1995 PC /JP95/02035 T
(71) Applicant: FUSO PHARMACEUTICAL INDUSTRIES LTD., Osaka-shi, Osaka 541 (JP)
(72) Inventor: WAKAMIYA, Nobutaka, Ibaraki-shi, Osaka 567 (JP)
(74) Representative: Towler, Philip Dean
(86) International application number: JP9600173
(87) International publication number: WO9707210

(57) **Abstract**

A recombinant conglutinin which contains a collagen region consisting of six amino acids containing two amino acid sequences Gly-Xaa-Xaa (SEQ ID NO:3, wherein Xaa stands for a protein-constituting amino acid), the neck region of natural conglutinin and the sugar chain recognition region of natural conglutinin, has an antiviral activity (virus neutralizing activity), and is expected to be applicable to drugs; and a process for detecting anti-influenza A virus activity of a mannose-binding protein (MBP) or a human mannose-binding protein (hMBP) involving the step of treating influenza A virus-infected cells with the MBP or hMBP and measuring the level of the suppression of the budding of the virus in the virus-infected cells. An MBP and an hMBP having an anti-influenza A virus activity are disclosed.

## Description

### [Technical Field]

The present invention relates to recombinant conglutinin having anti-virus activities (neutralization activities) which are expected to be applied to medicines and producing method thereof, and a method for detecting physiological activities of collectins.

### [Background Art]

Conglutinin is an animal lectin belonged to calcium-dependent mammalian C-type lectin family and existed in the bovine serum. Whole amino acids sequence (SEQ ID No.:1) had been analyzed by Lee et al., [Lee et al., J. Biol. Chem., Vol. 266, pp. 2715-2723, 1991].

C-type lectin comprises basic unit having the four unique regions of (1) N-terminal region contained much cysteine, (2) collagen-like region, (3) neck region and (4) carbohydrate recognition domain (CRD) [Malhortra et al., European Journal Immunology, Vol. 22, pp. 1437-1445, 1992].

Besides conglutinin, C-type lectin includes Mannan-Binding Proteins (MBP), Surfactant Protein A (SP-A) and surfactant Protein D (SP-D), and they are generally called as collectin.

In vertebrates, mechanisms involving specific antibody reaction and immune response through the cells are considered as a main host-defense system against inversion of the pathogenic bacteria. However, recently, non-specific immune response by these lectins seems that it may play an important role to neutralize and remove the various microorganisms in the puerile subjects having the maternal transmigration antibody and the undeveloped specific defence system [Super et al., Lancet, Vol. II, pp. 1236-1239, 1989].

Regarding the role of these lectins on biological defence in host organism, it is reported that infection will be easily spread by, for example, the reduction of the mannan-binding protein concentration in blood due to genetic mutation of the mannan-binding protein [Sumiya et al., Lancet, Vol.337, pp.1569-1570, 1991].

The present inventor once reported that the conglutinin and the mannan-binding protein inhibit infection and hemagglutination inhibition activity of H1 and H3 Type Influenza A Viruses (Wakamiya et al., Glycoconjugate J., Vol.8, p.235, 1991 ; Wakamiya et al., Biochem. Biophys. Res. Comm., Vol. 187, pp. 1270-1278, 1992).

Thereafter, the research group of the present inventor isolated cDNA clone encoding the conglutinin and found that there is the closer correlation between gene of the conglutinin and that from the various surfactant protein-D [Suzuki et al., Biochem. Biophys. Res. Comm., Vol. 191, pp.335-342, 1993].

Accordingly, the conglutinin have been expected as useful material for physiologically active medicine component, but amount of the conglutinin to be obtained from the bovine serum is less. Further, continuous production of the conglutinin is quite difficult because source thereof is completely depended on an animal body. Expression of the conglutinin in Escherichia coli by the genetic recombinant techniques had been tried to realize the large scale production of the conglutinin.

In such process, first of all, whole cDNA of the conglutinin was amplified by PCR (Polymerase Chain Reaction) method, then the amplified genes were introduced into the expression vector pRSET-A and were expressed with M13/T7 phage. The recombinant conglutinin obtained was analyzed. Although expression of the recombinant conglutinin had been confirmed, expressed amounts are less to be barely detected by Western blotting. This approach is inconvenient to the large-scale production of the conglutinin.

Similar methods had also been tried by using another expression vectors, but the same or less expression level had merely detected by any of the vectors. Anyway, an effective expression system have not been realized yet in the art. This seems due to difficulties in expressing the conglutinin because Escherichia coli does not possess proteins of the structure like collagen-like region. Further, yield of the conglutinin produced from an eukaryotic cells is little, and some of the conglutinin may sometimes have an inappropriate posttranscriptional modification.

As stated above, although the conglutinin have been expected as an useful medicine component, neither the natural source nor the genetic recombinant techniques could provide the large amount of the conglutinin.

### [Disclosure of Invention]

The present inventions are established to solve the aforenoted problems in the prior art, and they are based on the findings that large amount of the present recombinant conglutinin can be produced according to the previously noted expression system, wherein the recombinant conglutinin comprises (i) a part of the collagen region of the conglutinin consisting of 171 amino acids sequence (SEQ ID No.:2), namely, an extremely short collagen region consisting of six amino acids comprising two units of amino acids sequence of Gly-Xaa-Xaa (SEQ ID No.: 3; 2nd and 3rd amino acids are protein-constituting amino acid), (ii) the neck region and (iii) the carbohydrate recognition domain.

Despite that the recombinant conglutinin of the present invention comprises the extremely short collagen region, the neck region and the carbohydrate recognition domain, they maintain the similar activities to be expressed by the native conglutinin including the activities of the sugar binding specificities, conglutination activities depending on calcium, hemagglutination inhibition (HI) activities against Influenza A viruses, neutralization activities and viral growth (infection spread) inhibition activities.

Further, the method for detecting the physiological activities of the collectins can be used to detect the physiological activities of the collectins including the conglutinin by evaluating inhibition effects on budding of the viruses from the cells preinfected with viruses, in particular, with influenza A virus. According to this method, physiological activities of the collectins can exactly be detected even if they were once determined as inactive by the conventional detection method (e.g., detection by neutralization activities), physiological activities of the collectins would therefore be appropriately evaluated from different aspects. Further, the present detection method may provide a landmark to determine a preferable use of the collectins.

### [Brief Description of Drawings]

Figure 1 shows a vector to transform subjects with the recombinant conglutinin DNA;
Figure 2 shows a result of SDS-PAGE on the recombinant fusion conglutinin;
Figure 3 shows a result of absorbance, CBB staining and Western blotting on each fraction by Mannan-Sepharose Affinity Chromatography;
Figure 4 shows a result of SDS-PAGE on the fractions treated with bis(sulfosuccinymidil)suberate;
Figure 5 (a), (b) and (c) show a result of gel filtration chromatography on the recombinant conglutinin;
Figure 6 is a graph showing binding-activity between mannan and the recombinant conglutinin or that containing 20mM N-acetylglucosamine;
Figure 7 is a graph showing binding-activity between mannan and the recombinant conglutinin or that containing 10mM EDTA;
Figure 8 shows conglutination activities on the recombinant conglutinin and the native conglutinin with mictotiter plate assay system;
Figure 9 shows hemagglutination inhibition (HI) activities on the native conglutinin and the recombinant conglutinin;
Figure 10 (a) and (b) show viral growth (infection spread) inhibition activities on the recombinant conglutinin (rBKg-CRD);
Figure 11 (a) and (b) show viral growth (infection spread) inhibition activities on the recombinant conglutinin (rBKg-CRD);
Figure 12 (a) and (b) illustratively show detection mechanism according to the conventional evaluation method on neutralization acitivities and the present invention;
Figure 13 shows viral growth inhibition by the human mannan-binding protein (hMBP);
Figure 14 is a graph showing viral growth inhibition by the human mannan-binding protein (hMBP); and
Figure 15 shows viral growth inhibition by the recombinant conglutinin (rBKg-CRD), hMBP and rabbit MBP.

### [Best Mode for Carrying Out the Invention]

The recombinant conglutinin of the present inventions will be explained in detail along with the following Examples, but, as a matter of course, scope of the present inventions should not be limited based on the disclosures of the Examples.

Examples are consisting of, expression of conglutinin fragments in Escherichia coli (Example 1), structural analysis on the recombinant conglutinin (Example 2), evaluation on sugar binding activities of the recombinant conglutinin and the native conglutinin (Example 3), evaluation on conglutimation activities of the recombinant conglutinin (Example 4), activities on hemagglutination inhibition (HI) (Example 5), neutralization activities (Example 6), activities on viral growth (infection spread) inhibition (Example 7), and detection of physiological activities by collectins (Example 8).

### Example 1: Expression of Conglutinin Fragments in Escherichia coli

### (1) Preparation of Conglutinin DNA Fragments with RT-PCR

In accordance with the method by Suzuki et al., (Biochem. Biophys. Res. Comm., Vol.191, pp.335-342, 1993), primers for PCR containing the following sequences were designed based on cDNA of the bovinbe conglutinin and were synthesized. Each of these primers has cleavage sites of the restriction enzymes XhoI and EcoRI.
5'-GGCTCGAGGGGGAGAGTGGGCTTGCAGA-3' (SEQ ID No.:4)
5'-GGGAATTCTCAAAACTCGCAGATCACAA-3' (SEQ ID No.:5)

50 µl of reaction mixture was used as a sample containing 1×buffer, 1 µM primers, 200 µM dNTPs, 1U Deep Bent DNA polymerasae (NewEngland Bio labs) and 10ng cDNA. Using PCR reactor of Atto (Zymoreactor (Registered Trade Mark)), PCR was performed for 35 cycles, each cycle of which consists of denaturation at 92°C for one minute, annealing at 60°C for one minute and elogation at 72°C for two minutes. PCR products of 497bp was produced.

### (2) Preparation of Transformants by Conglutinin Fragments

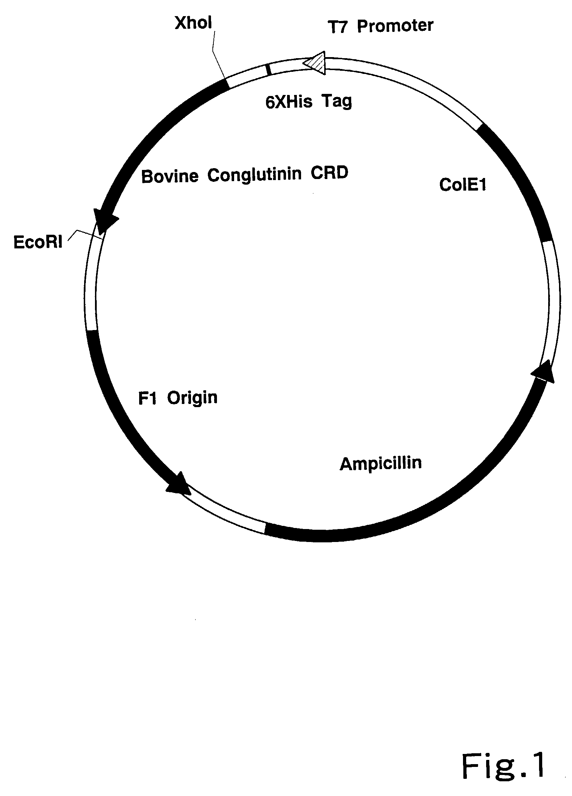

PCR products of Example 1(1) were digested with the restriction enzymes XhoI and EcoRI, then were inserted into the expression vector pRSET-A (Invitrogen) with DNA ligation kit (Takara Shuzo). Ligation solution was then transfected into Escherichia coli JM109 and transformants was obtained that have the conglutinin DNA fragments corresponding to 631bp through 1113bp of the native conglutinin DNA (Figure 1).

Sequences of these fragments were corresponding to 191st through 351st amino acids of native conglutinin, namely, PCR exactly amplified the sequences having the short collagen region, the neck region and the carbohydrate recognition domain. Further, there was no error in the PCR reaction. Accordingly, desirous stable transformants were obtained which can remarkably produce such conglutinin DNA fragments.

### (3) Expression and Purification of Recombinant Conglutinin Proteins

Transformed single colony containing whole insert (conglutinin DNA fragment) was incubated overnight at 37°C on SOB medium (containing 50 µg/l ampicillin). 1.2ml of culture solution was inoculated onto 200ml SOB medium (containing 50 µg/l ampicillin) and cells were allowed to grow to be approximately 0.3 of OD₆₀₀ₙₘ. Isopropyl-1-thio-β-D-galactoside (IPTG) was then added to become final concentration of 1 mM and the culture was grown for additional one hour. The cells were infected at MOI 5 pfu/cell with M13 phage containing T7 Δ RNA polymerase gene driven by the Escherichia coli lactose promoter and incubated for another three hours. Bacteria was collected by centrifugating the culture solution at 3,000g for 15 minutes.

Pellets of bacteria were suspended in 20ml Buffer A (guanidine chloride 6M, sodium phosphate 20mM, sodium chloride 500mM, pH 7.8) and were lysed with sonication (15 seconds, power 70%, 10 times). After centrifugation at 43,000g for 30 minutes, Nickel-NTA agarose (Qiagen) was added to the supernatant and they were left for 15 minutes.

Products were poured into a column. The column was washed with TBS/NT solution (Tris-HCl 20mM, sodium chloride 140mM, 0.05% sodium azide, 0.05% Tween 20 (Registered Trade Mark), pH7.4) and further with TBS/NTC solution (TBS/NT solution containing 5 mM calcium chloride). Fusion proteins were eluted with TBS/NTC solution containing 0.5mM imidazole. Eluted solution was dialyzed three times against 1,000 times volume of TBS/NTC solution. After dialysis and centrifugation of the samples, the supernatant was poured into the Mannan-Sepharose Column (Affinity Column prepared by binding Mannan (Sigma) to CNBr activated Sepharose 4B (Pharmacia)). After washing with the TBS/NTC solution, proteins in the column were eluted with TBS/NTC solution containing 5mM N-acetylglucosamine.

Purity of the recombinant conglutinin produced was determined by SDS-PAGE or Western blotting as noted later.

### (4) SDS-PAGE

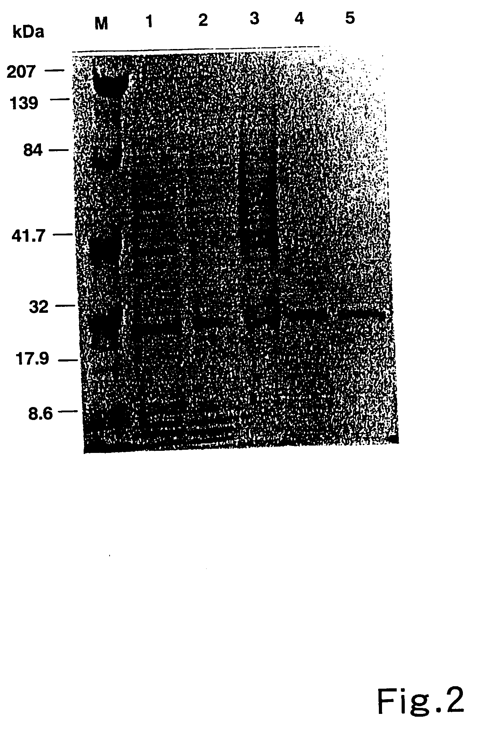

In SDS-PAGE, polyacrylamide gel having the 4-20% concentration gradient was employed. Polypeptide was stained with 1% Coomassie Brilliant Blue (CBB). Results were shown in Figure 2. In Figure 2, Lane M is standard proteins, Lane 1 is a lysate of whole cell, Lane 2 is a soluble fraction by guanidine chloride, Lane 3 is an insoluble fraction by guanidine chloride, Lane 4 is an eluted fraction from nickel-agarose column and Lane 5 is an eluted fraction from Mannan-Sepharose column. Although the molecular weight of the recombinant fusion conglutinin deduced from the amino acids sequences was 22.5kDa, the molecular weight analyzed by SDS-PAGE was 27kDa. Despite the digestion of the recombinant conglutinin with an enterokinase was not well, N-termimal amino acids sequence in the digested minor recombinant conglutinin was coincided with that of the matured conglutinin.

### (5) Mannan-Sephalose Affinity Chromatography, SDS-PAGE and Western blotting on Each Fraction

Eluted fractions from Mannan-Sephalose Column were analyzed by SDS-PAGE under the conditions of polyacrylamide gel having 4-20% concentration gradient and 1% Coomassie Brilliant Blue staining. Proteins were transferred to nitrocellulose membrane and were incubated with 2,000-fold diluted rabbit anti-conglutinin serum. Then, they were reacted with anti-rabbit IgG-conjugated biotin (Vector) and finally with alkaline phosphatase-conjugated streptavidin (BRL). NBT/BCIP (BRL) was used as substrate for the alkaline phosphatase.

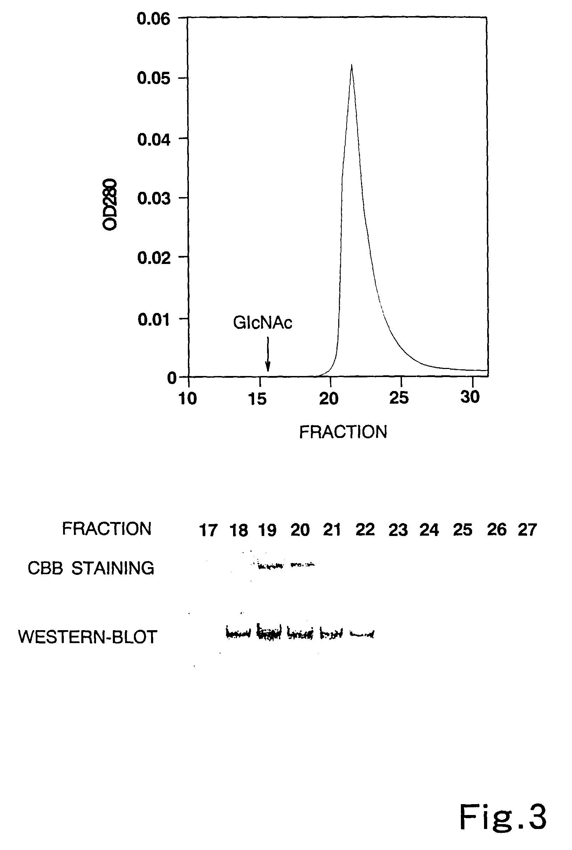

Elution pattern was shown in Figure 3. Like the native conglutinin, the recombinant conglutinin had been eluted with 5mM N-acetylglucosamine. This is to demonstrate that these fusion proteins have strong affinity against mannan. Final yield of the purified recombinant conglutinin was 2.8mg per one liter of culture solution of Escherichia coli.

### Example 2: Structural Analysis of Recombinant Conglutinin

### (1) Crosslinking of Recombinant Fusion Conglutinin

Native conglutinin consists of enneamer (9 mer) through octadecamer (18 mer) polypeptides (Kawasaki et al., Arch. Biochem. Biophys., vol. 305, pp.533-540, 1993). Molecular weight of the biggest polymer is approximately 1,000kDa, and structure of the typical polymer forms four cross-shaped trimer (Strang et al., Biochem. J., Vol.234, 381-389, 1986). Then, the structure of the recombinant conglutinin had also been analyzed.

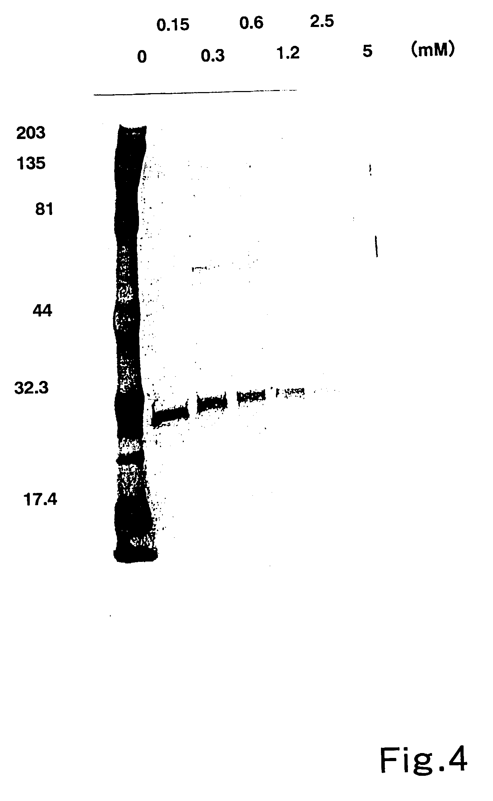

The recombinant conglutinin proteins were dissolved with the PBS buffer containing 10mM calcium chloride in the concentration of 22.8 µg/ml. Samples were treated at 37°C for 20 minutes with 0mM, 0.15mM, 0.3 mM, 0.6mM, 1.2mM, 2.5mM and 5mM bis(sulfosuccinymidil)suberate, and were analyzed with SDS-PAGE of 4-20% concentration gradient. Results were shown in Figure 4. It was confirmed from the results of Figure 4 that the recombinant fusion conglutinin consists of the monomer and the trimer having the molecular weight of 27kDa.

### (2) Gel Filtration Chromatography

Purified recombinant conglutinin was applied to Superose 6 (Pharmacia) at a flow rate of 0.5 ml/minute with TBS buffer containing 10mM EDTA, pH 8.0. Then, 40 µg of the recombinant conglutinin was applied to this column. Fractions were monitored at 280nm. Amount of the collected recombinant proteins were assayed with Coomassie Proteins Assay Reagent (Pierce).

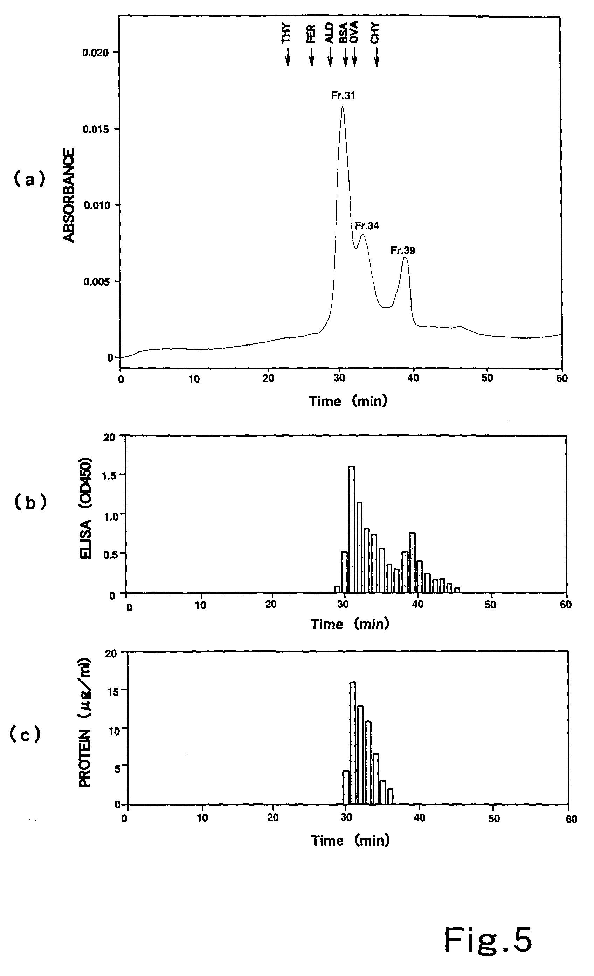

Each fraction was then applied to Sandwitch ELISA System employing the following anti-bovine conglutinin rabbit serum. Standard Molecular Weight Kit of Pharmacia (thyroglobulin (THY), ferritin (FER), catalase, aldolase (ALD), albumin (BSA), ovalbumin (OVA), chymotrypsinogen A (CHY), ribonuclease A) was used to calibrate the column. As shown in Figure 5, three major peaks of 94kDa (31st fraction), 39kDa (34th fraction) and 4.6kDa (39th fraction) had been found. However, no protein was detected in the 39th fraction by a quantitive analysis. Fraction of 4.6kDa had not been stained in the silver staining of SDS gels. This fraction was identified as non-peptide by the ultraviolet absorbance at 200mm and 280mm.

Based on the above results, it is apparent that the conglutinin may form trimer without the collagen region. Further, these results correspond to the facts that, like the conglutinin, the recombinant human mannan-binding protein or bovine lung surfactant protein D respectively belonged to C-type lectin forms trimer through the neck region without the collagen region (Sheriff et al., Nature Struct. Biol., Vol.1, pp.789-794, 1994; Hoppe et al., FEBS Lett., Vol.344, pp. 191-195, 1994).

### Example 3: Sugar Binding Activities by Recombinant Conglutinin and Native Conglutinin

### (1) Sugar Binding Activities

Microtiter Plates were coated with 100 µl coating buffer (15mM sodium carbonate, 35mM sodium hydrogencarbonate, 0.05% sodium azide, pH9.6) containing yeast mannan (10 µg/ml) at 4°C overnight. After each treatment step, the plates were washed three times with TBS/NTC solution (20mM Tris-HCl, 140mM sodium chloride, 0.05% sodium azide, 0.05% Tween 20 (Registered Trade Mark), pH 7.4, 5mM calcium chloride). After completing the coating of the plates, the plates were treated and blocked with TBS/NTC solution containing 1% bovine serum albumin at room temperature for one hour.

Single dilution (0, 1, 10, 100 and 1,000ng/ml) of the recombinant conglutinin or the native conglutinin, or mix dilution of the various sugars and such single dilution were added to TBS/NTC or TBS/NTC containing 20mM N-acetyl-D-glucosamine (A) or 10mM EDTA.

Rabbit anti-native conglutinin serum and goat anti-rabbit IgG horseradish peroxidase conjugates (Bio-Rad) respectively 1,000 or 2,000-fold diluted with TBS/NTCB were added thereto and they were incubated at 37°C for one hour. Finally, 100 µl of TMB substrates (TMB Microwell Peroxidase Substrates System; KPL) was added to each well. Before or after the addition of 100 µl of 1M phosphoric acid, absorbance at 450 or 655nm was measured (TiterTech MultiScan Plus MKII Plate Reader; Flow Rubs). Then, evaluation on sugar inhibitting activities were performed according to the method of Lu et al., (Biochem. J., Vol.,284, pp.795-802, 1992) employing this ELISA system.

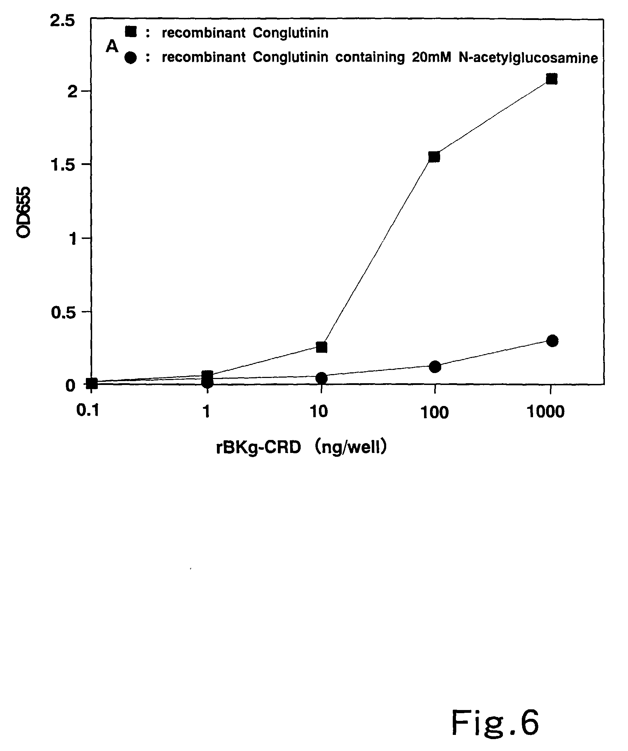

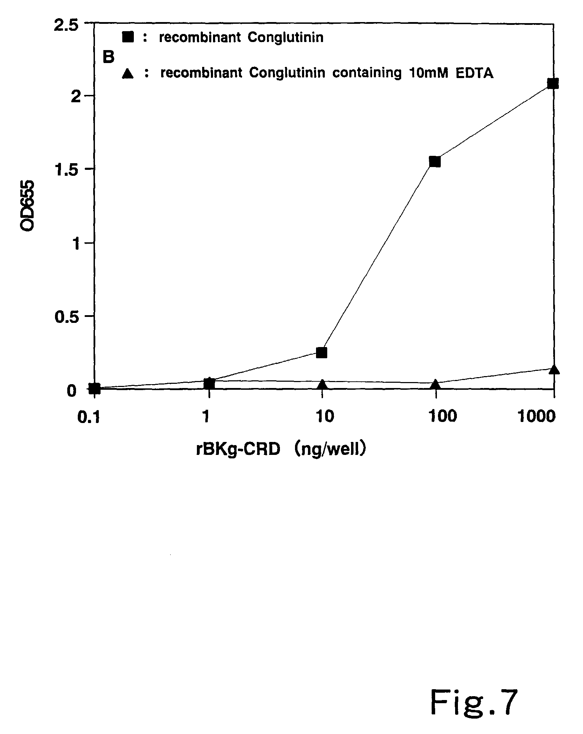

After coating the microtiter plates with yeast mannan (1 µg/well), the recombinant conglutinins were reacted with sugars. Sugar binding specificity (I₅₀) was shown as sugar concentration to halve binding activities. Results are shown in Table 1. Obviously from Table 1, sugar binding activities with the recombinant conglutinin are substantially same to that of the native conglutinin. Then, as shown in Figures 6 and 7, like the native conglutinin, binding activities of the recombinant conglutinin were depended on calcium ion. Further, these binding activities were inhibited by N-acetylglucosamine. On the other hand, tags of histidine fused to the recombinant conglutinin were not involved in the binding activities to mannan and binding specificities.

**TABLE 1**

| Sugar Binding Specificities on Recombinant Conglutinin and Native Conglutinin | | |
|---|---|---|
| | I₅₀(mM)* | |
| | Recombinant Conglutinin | Native Conglutinin** |
| N-Acetyl-D-Glucosamine | 0.65 | 1.4 |
| Fucose | | 41.5 |
| L-Fucose | 37.8 | |
| D-Fucose | 55.3 | |
| D-Mannose | 12.3 | 19.5 |
| Maltose | 25.8 | 49.0 |
| N-Acetyl-D-Mannosamine | 26.6 | |
| Glucose | 39.5 | 41.5 |
| Galactose | 46.8 | >100 |
| N-Acetyl-D-Galactosamine | ∞*** | |
| Lactose | >100 | ∞*** |

| | | |
|---|---|---|
| ∗: Sugar concentration to halve binding activity with mannan. | | |
| ∗∗: Lu, J. et al., Biochem. J., vol. 284, pp.795-802 (1992) | | |
| ∗∗∗: Inhibition-activity was not detected. | | |

### Example 4: Conglutination Activities by Recombinant Conglutinin

Conglutination activities by the recombinant conglutinin and the native conglutinin were evaluated by Microtiter plate assay system. Sheep erythrocyte cells with iC3b were prepared according to the method of Wakamiya et al., (Biochem. Biophys. Res. Comm., Vol.187, pp.1270-1278, 1992). Namely, 1% sheep erythrocyte cells with iC3b were prepared by priming with a mixture of ten-fold diluted fresh horse serum and equivalent amount of anti-Forssmann antibody, and incubated at 37°C for ten minutes.

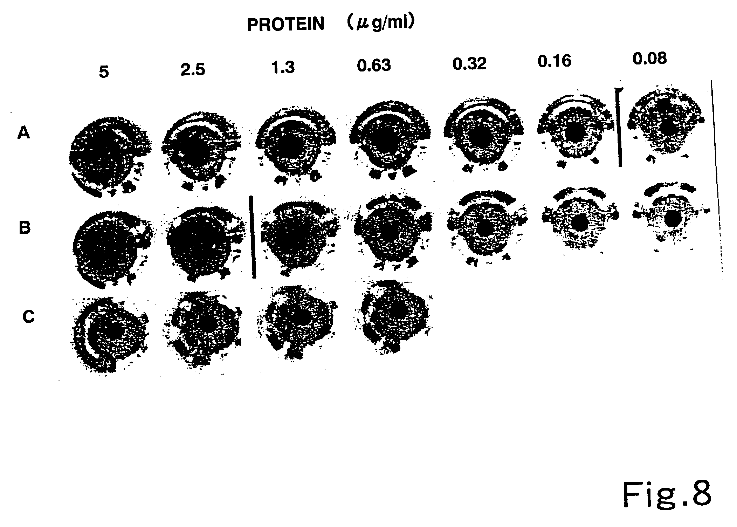

50 µl of 1% sheep erythrocyte cells with iC3b and 50 µl of the recombinant conglutinin or 50 µl of the native conglutinin was added to the raw veronal buffer or the veronal buffer containing 30mM N-acetylglucosamine. Then, they were incubated at 37°C and the conglutination activities thereon were detected. The lowest concentration of the proteins to cause agglutination is regarded as titer of conglutination, then the results are shown in Figure 8. In Figure 8, Lane A is the native conglutinin, Lane B is the recombinant conglutinin and Lane C is the recombinant conglutinin containing 30mM N-acetylglucosamine. Titer of conglutination on the native conglutinin was 0.16 µg/ml, while that of the recombinant conglutinin was 1.3-2.5 µg /ml. Such activities were completely inhibited by 30mM N-acetylglucosamine (GlcNAc).

### Example 5: Hemagglutination Inhibition (HI) Activities

### (1) Viruses

Influenza A virus, namely, Influenza A virus A/Ibaraki/1/90 (H3N2: Influenza A virus (A-Hong Kong)), A/Osaka/869/95 (H3N2), A/Beijing/352/89 (H3N2), A/Adachi/1/57 (H2N2) and A/Suita/1/89 (H1N1:Influenza A virus (A-U.S.S.R.)) were used to evaluate Hemagglutination Inhibition (HI) Activities. Viruses were proliferated with CAM (chorioallantoic membrane) according to the standard method and were stored at -70°C until use. As a growth medium for the viruses, Eagle MEM medium containing 3% vitamin for tissue cultures, 0.2% albumin, 0.1% glucose and 0.2ng/ml acetylated trypsine was used.

### (2) Hemagglutination Inhibition (HI) Activities By Recombinant Conglutinin

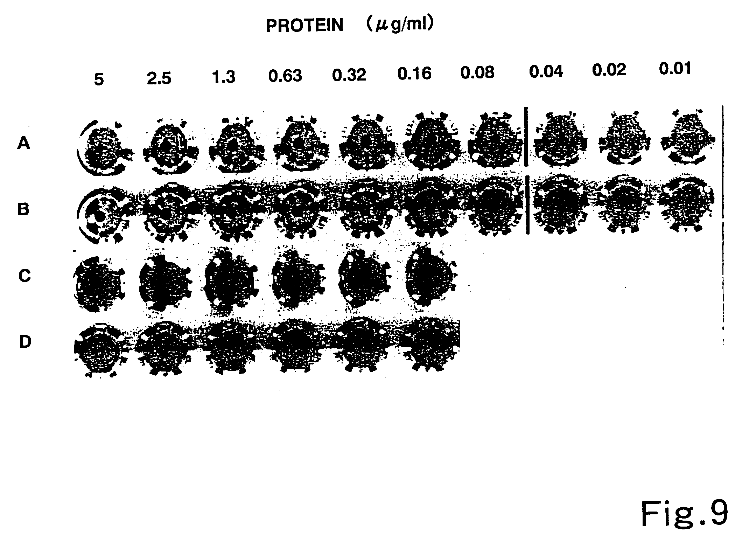

In accordance with the method of Okuno et al., (J. Clin. Microbiol., Vol.28, pp.1308-1313, 1990), experiments were performed by 96-well microtiter plates with 1% chick's erythrocytes. The ether-treated virus antigens from an hen egg antigen was used. No additive had been added to mixed cultivation solution of TBS/C (TBS solution containing 5mM sodium chloride) except for 30mM N-acetylglucosamine or 10mM EDTA. After incubation at room temperature for one hour, effects on the recombinant conglutinin fragments (rBKg-CRD) against viral hemagglutination on chick's erythrocytes were observed. Results are shown in Table 2. Results on Influenza A virus A/Ibaraki/ 1/90 are shown in Figure 9. In Figure 9, Lane A is the native conglutinin, and Lanes B, C and D are directed to the recombinant conglutinin fragments, in which the Lane B is no additives, Lane C is added thereto 30mM N-acetylglucosamine and Lane D is added threreto 10mM EDTA.

**TABLE 2**

| Expression Concentration (µg/ml) on Hemagglutination Inhibition (HI) by Recombinant Conglutinin and Native Conglutinin | | |
|---|---|---|
| Virus | Recombinant Conglutinin | Native Conglutinin |
| A/Suita/1/89(H1N1) | 0.15-0.3 | 0.08 |
| A/Adachi/1/57(H2N2) | >5 | >5 |
| A/Ibaraki/1/90(H3N2) | 0.08-0.3 | 0.08 |
| A/Beijing/352/89(H3N2) | 0.3 | nt∗ |
| A/Osaka/869/95(H3N2) | 0.15 | nt∗ |

| | | |
|---|---|---|
| ∗ Not tested. | | |

Hemagglutination Inhibition (HI) activities were depended on dosages and calcium. Further, Hemagglutination Inhibition (HI) activities of the recombinant conglutinin is substantially same level to the titer of the native conglutinin, rat surfactant protein D, human surfactant protein D (Hartshorn et al., J. Clin. Invest., Vol.,94, pp.311-319, 1994).

### Example 6: Neutralization Activities

### (1) Viruses

Influenza A virus, namely, Influenza A virus A/Ibaraki/1/90 (H3N2: Influenza A virus (A-Hong Kong)) and A/Suita/1/89 (H1N1:Influenza A virus (A-U.S.S.R.)) were used.

### (2) Neutralization Activities

Neutralization activities were evaluated according to the method of Okuno et al., (J. Clin. Microbiol., Vol.28, pp.1308-1313, 1990). Influenza viruses and the recombinant conglutinin were mixed on 96-well microtiter plate, and the mixtures were incubated for several days in Madin-Darby Canine Kidney (MDCK) cells grown in the Eagle MEM medium containing 10% fetal bovine serum. Then, the neutralization activities by the various conglutinin were detected. The focuses infected by Influenza viruses were detected by anti-influenza virus mouse monoclonal antibody, anti-mouse IgG goat serum, and peroxidase anti-peroxidase (PAP) staining system.

Neutralization activities by the recombinant conglutinin and the native conglutinin were shown in the following Table 3. Neutralization titer was shown as concentration to inhibit half (50%) of the infection.

**TABLE 3**

| Neutralization Titer (µg/ml) of Recombinant Conglutinin and Native Conglutinin on Influenza A Viruses | | |
|---|---|---|
| Virus | Recombinant Conglutinin | Native Conglutinin |
| A/Ibaraki/1/90(H3N2) | 0.22-0.63 | 0.08 |
| A/Suita/1/89(H1N1) | 0.31 | nt∗ |

| | | |
|---|---|---|
| ∗ Not tested. | | |

### Example 7: Viral Growth (Infection Spread) Inhibition Activities

### (1) Viruses

Influenza A virus, namely, Influenza A virus A/Ibaraki/1/90 (H3N2: Influenza A virus (A Hong Kong)) was used.

### (2) Viral Growth (Infection Spread) Inhibition Activities

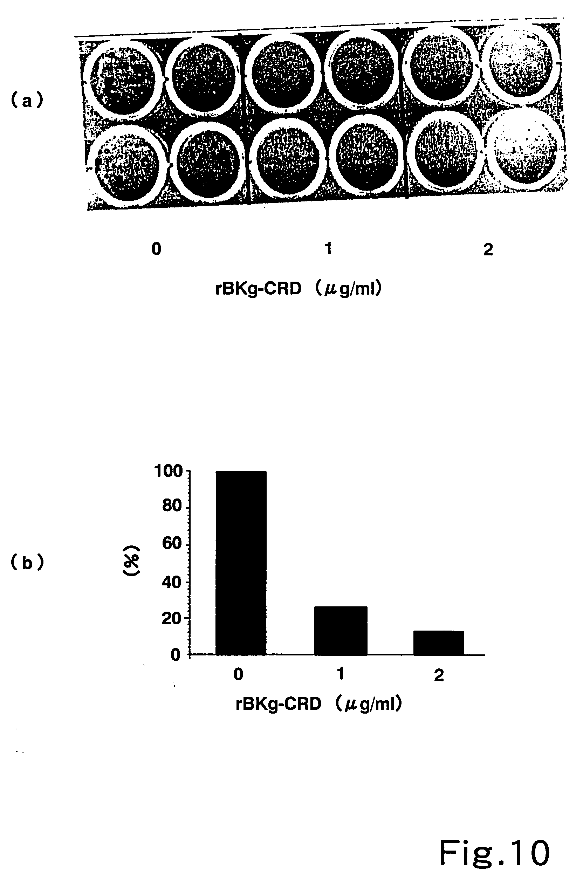

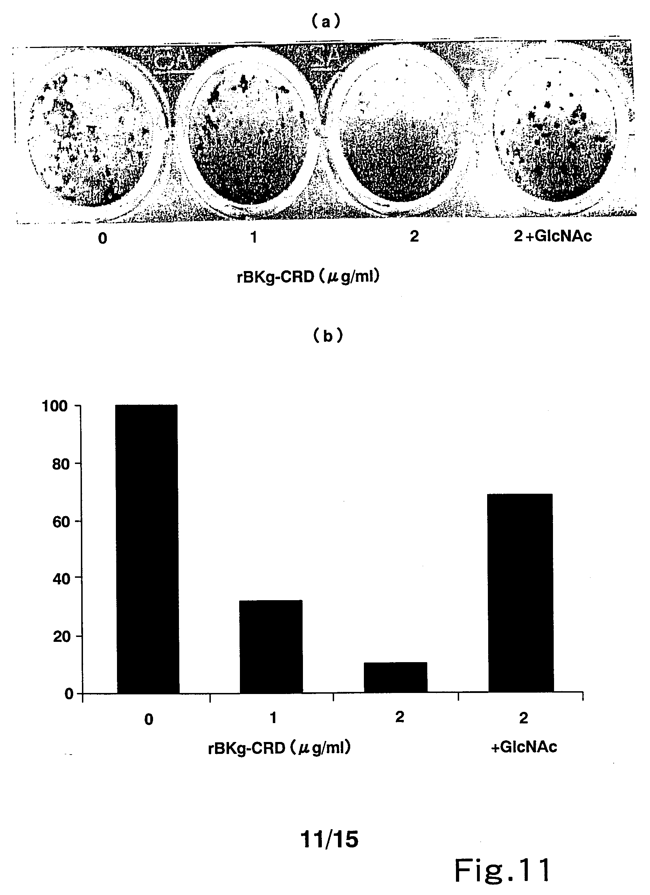

Influenza viruses were inoculated onto Madin-Darby Canine Kidney (MDCK) cells in which the cells were cultured in 24-well microtiter plates with the Eagle MEM medium containing 10% fetal bovine serum. After washing the cells, they were incubated for three days in the growth medium for the influenza virus containing 0.5% tragacanth gum (Sigma) and any of 0, 1 and 2 µg/ml recombinant conglutinin. Like the procedure in the experiment on Neutralization Activities referred to in Example 6 (2), gross areas of the virus-infected focus were detected by PAP staining. Samples without the recombinant conglutinin were used as control. Results are shown in Figures 10 (a) and (b). Obviously from the results in Figures 10 (a) and (b), the recombinant conglutinin reduces the area of the infected focus in a dose dependent manner and inhibits the viral growth. This effect was inhibited by 2 µg/ml N-acetylglucosamine (GlcNAc) (Figure 11).

### Example 8: Detection of Physiological Activities of Collectins

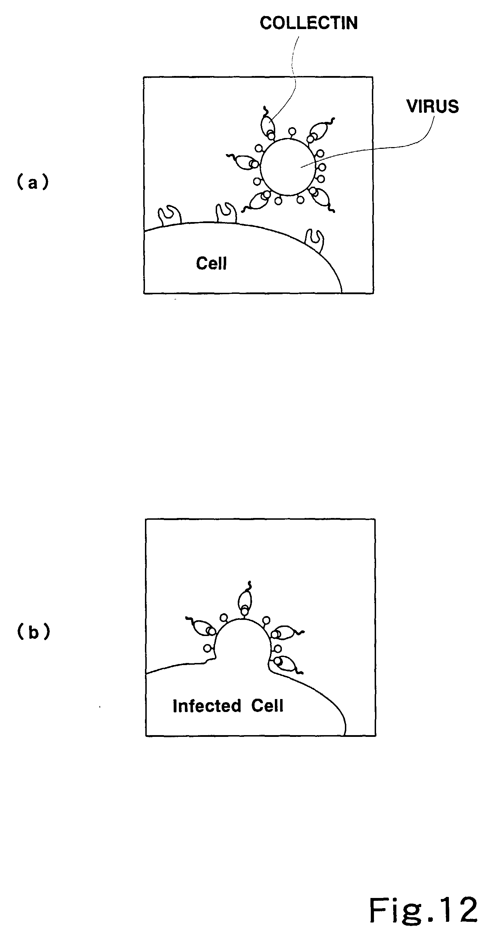

Detection method for the physiological activities of the collectins was constructed. The method is to evaluate the inhibition-effects on budding of the viruses from the cells preinfected with virus. The conventional method for detecting the physiological activities (e.g., detection on neutralization activities referred to in Example 6) comprises steps of contacting the collectins with viruses, infecting the cells with viruses of not binding to the collectins, and determing infection level by the binding activities between viruses and collectins, namely, neutralization activity (Figure 12 (a)). In contrast thereto, the present method detects the physiological activities of the collectins by evaluating the inhibition-effects on budding of the viruses from the cells preinfected with virus (Figure 12 (b)).

Physiological activities against Influenza A viruses were evaluated in accordance with the evaluation method on Hemagglutination Inhibition (HI) Activities according to Example 5, the evaluation method on Neutralization Activities according to Example 6, the evaluation on Hemagglutinin (HA) Activities by Western blotting, and the present method referred to in Example 7. Further, the neutralization activities against Influenza A viruses by the various collectins were also evaluated by the method referred to in Example 6. Results were shown in the following Tables 4 and 5.

**TABLE 4**

| | HI Activity | Neutralization Activity | HA Binding | Present Method |
|---|---|---|---|---|
| Bovine Conglutinin | | | | |
| Native | + | + | + | - |
| Recombinant | + | + | + | + |

| hMBP | | | | |
|---|---|---|---|---|
| Native | + | - | - | + |
| Recombinant | - | - | - | - |

| Human Surfactant Protein (hSP-D) | | | | |
|---|---|---|---|---|
| Native | + | + | + | - |
| Recombinant | - | - | ± | - |
| Abbreviation: +, Presence of (Binding) Activity -, Absence of (Binding) Activity | | | | |

**TABLE 5**

| | Neutralization Activity (Minimun Protein Concentration) |
|---|---|
| Human MBP | - (>5 µg/ml) |
| Rat MBP | ± (3 µg/ml) |
| Murine MBP | - (>8 µg/ml) |
| Rabbit MBP | + (0.07 µg/ml) |
| Bovine Conglutinin | + (0.09 µg/ml) |
| cf: Minimun Protein Concentration is necessary concentration to reduce infection scale down to 50% or less of control infected model. | |

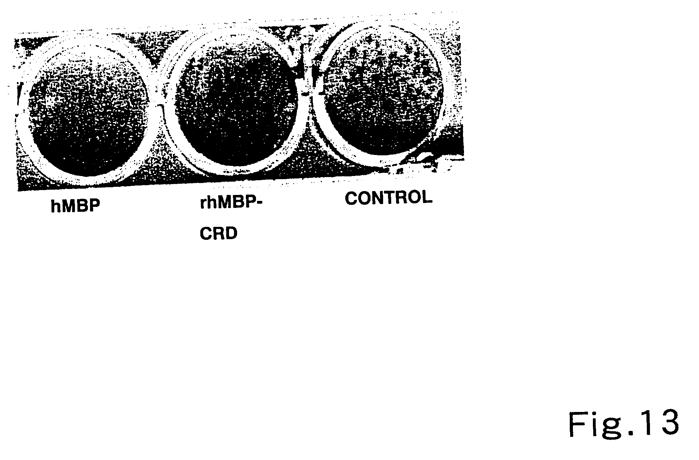

Obviously from the results of Tables 4 and 5, the conventional method did not detect the neutralization activities against Influenza viruses by either the native hMBP or the recombinant hMBP. In contrast thereto, the present method surprising indicating the facts on hMBP that the native hMBP inhibited the viral growth (the infection spread). Then, inhibition effects on budding of viruses were evaluated on the native hMBP and the recombinant hMBP-CRD in accordance with the present method referred to in Example 7 by using, as a control, buffer solution without collectins. Results were shown in Table 6 below and Figure 13.

**TABLE 6**

| | Viral Growth Inhibition Activity (Minimum Protein Concentration) |
|---|---|
| native hMBP | + (0.05 µg/ml) |
| recombinant hMBP-CRD | - (>2.5 µg/ml) |
| cf: Minimun Protein Concentration is necessary concentration to reduce infection scale down to 50% or less of control infected model. | |

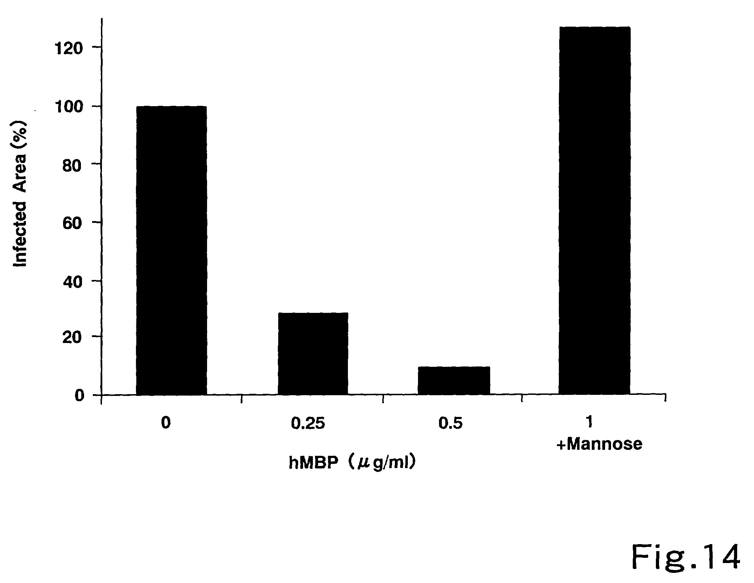

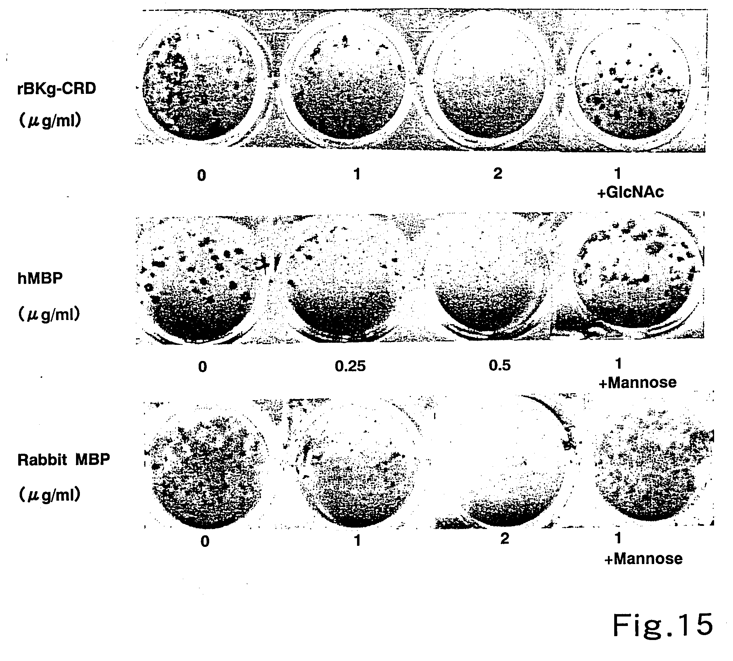

Results shown in Table 6 above and Figure 13 demonstrated that the native hMBP inhibits growth (infection spread) of the viruses. The following experiments were performed to further demonstrate such new findings on the functions by hMBP. Influenza A viruses were inoculated onto Madin-Darby Canine Kidney (MDCK) cells in which the cells were cultured in 24-well microtiter plates with the Eagle MEM medium containing 10% fetal bovine serum. After washing the cells, they were incubated for three days in the growth medium for Influenza A viruses containing 0.5% tragacanth gum (Sigma) and any of 0, 0.25 and 0.5 µg/ml hMBP. Like the procedure in the experiment on Neutralization Activities referred to in Example 6 (2), gross areas of the virus-infected focus were detected by PAP staining. Samples containing 1 µg/ml N-acetylglucosamine (GlcNAc) were used as control. Results are shown in Figure 14. Obviously from the results in Figure 14, hMBP reduced the area of the infected focus in a dose dependent manner and inhibited the viral growth. Such effects were also found in the recombinant conglutinin (rBKg-CRD), hMBP and rabbit MBP, when the physiological activities against Influenza A virus were evaluated along with the previously noted method (Figure 15). In such evaluations, 1 µg/ml N-acetylglucosamine (GlcNAc) and 1 µg/ml mannose were employed as a control respectively for rBKg-CRD and for hMBP and rabbit MBP.

### [Industrial Applicability]

According to the present invention, means for artificially producing the large amount of the recombinant conglutinin can be realized wherein the recombinant conglutinin maintain the equivalent physiological activities to be expressed by the native conglutinin obtained with extremely low yield from the animal (bovine). Since the recombinant conglutinin of the present invention maintains the equivalent physiological activities to be expressed by the native conglutinin, its usefulness as a medicine will also be expected. Then, the recombinant conglutinin of the present invention is a part of the native conglutinin and have less molecular weight in comparison it with the native conglutinin, therefore, purification thereof will become smoother and advantages may offer in its manufacturing process.

In addition thereto, according to the present invention, novel method for detecting physiological activities of the collectins is also provided, then, physiological activities of the collectins can be evaluated from different aspects in combination with another conventional detection method. Further, the present detection method may provide a landmark to determine a preferable use of the collectins.

SEQUENCE LISTING

SEQ ID No.:1

SEQUENCE LENGTH: 351

SEQUENCE TYPE: amino acid

TOPOLOGY: linear

MOLECULAR TYPE: protein

SEQUENCE:

Ala Glu Met Thr Thr Phe Ser Gln Lys Ile Leu Ala Asn Ala Cys Thr

1               5                   10                  15

Leu Val Met Cys Ser Pro Leu Glu Ser Gly Leu Pro Gly His Asp Gly

            20                  25                  30

Gln Asp Gly Arg Glu Cys Pro His Gly Glu Lys Gly Asp Pro Gly Ser

        35                  40                  45

Pro Gly Pro Ala Gly Arg Ala Gly Arg Pro Gly Trp Val Gly Pro Ile

    50                  55                  60

Gly Pro Lys Gly Asp Asn Gly Phe Val Gly Glu Pro Gly Pro Lys Gly

65                  70                  75                  80

Asp Thr Gly Pro Arg Gly Pro Pro Gly Met Pro Gly Pro Ala Gly Arg

                85                  90                  95

Glu Gly Pro Ser Gly Lys Gln Gly Ser Met Gly Pro Pro Gly Thr Pro

            100                 105                 110

Gly Pro Lys Gly Glu Thr Gly Pro Lys Gly Gly Val Gly Ala Pro Gly

        115                 120                 125

Ile Gln Gly Phe Pro Gly Pro Ser Gly Leu Lys Gly Glu Lys Gly Ala

    130                 135                 140

Pro Gly Glu Thr Gly Ala Pro Gly Arg Ala Gly Val Thr Gly Pro Ser

145                 150                 155                 160

Gly Ala Ile Gly Pro Gln Gly Pro Ser Gly Ala Arg Gly Pro Pro Gly

                165                 170                 175
Leu Lys Gly Asp Arg Gly Asp Pro Gly Glu Thr Gly Ala Ser Gly Glu
            180                 185                 190
Ser Gly Leu Ala Glu Val Asn Ala Leu Lys Gln Arg Val Thr Ile Leu
        195                 200                 205
Asp Gly His Leu Arg Arg Phe Gln Asn Ala Phe Ser Gln Tyr Lys Lys
    210                 215                 220
Ala Val Leu Phe Pro Asp Gly Gln Ala Val Gly Glu Lys Ile Phe Lys
225                 230                 235                 240
Thr Ala Gly Ala Val Lys Ser Tyr Ser Asp Ala Glu Gln Leu Cys Arg
                245                 250                 255
Glu Ala Lys Gly Gln Leu Ala Ser Pro Arg Ser Ser Ala Glu Asn Glu
            260                 265                 270
Ala Val Thr Gln Met Val Arg Ala Gln Glu Lys Asn Ala Tyr Leu Ser
        275                 280                 285
Met Asn Asp Ile Ser Thr Glu Gly Arg Phe Thr Tyr Pro Thr Gly Glu
    290                 295                 300
Ile Leu Val Tyr Ser Asn Trp Ala Asp Gly Glu Pro Asn Asn Ser Asp
305                 310                 315                 320
Glu Gly Gln Pro Glu Asn Cys Val Glu Ile Phe Pro Asp Gly Lys Trp
                325                 330                 335
Asn Asp Val Pro Cys Ser Lys Gln Leu Leu Val Ile Cys Glu Phe
            340                 345                 350

SEQ ID No.:2
SEQUENCE LENGTH: 171
SEQUENCE TYPE: amino acid
TOPOLOGY: linear
MOLECULAR TYPE: protein

SEQUENCE:
Gly Leu Pro Gly His Asp Gly Gln Asp Gly Arg Glu Cys Pro His Gly
1               5                   10                  15
Glu Lys Gly Asp Pro Gly Ser Pro Gly Pro Ala Gly Arg Ala Gly Arg
            20                  25                  30
Pro Gly Trp Val Gly Pro Ile Gly Pro Lys Gly Asp Asn Gly Phe Val
        35                  40                  45
Gly Glu Pro Gly Pro Lys Gly Asp Thr Gly Pro Arg Gly Pro Pro Gly
    50                  55                  60
Met Pro Gly Pro Ala Gly Arg Glu Gly Pro Ser Gly Lys Gln Gly Ser
65                  70                  75                  80
Met Gly Pro Pro Gly Thr Pro Gly Pro Lys Gly Glu Thr Gly Pro Lys
                85                  90                  95
Gly Gly Val Gly Ala Pro Gly Ile Gln Gly Phe Pro Gly Pro Ser Gly
            100                 105                 110
Leu Lys Gly Glu Lys Gly Ala Pro Gly Glu Thr Gly Ala Pro Gly Arg
        115                 120                 125
Ala Gly Val Thr Gly Pro Ser Gly Ala Ile Gly Pro Gln Gly Pro Ser
    130                 135                 140
Gly Ala Arg Gly Pro Pro Gly Leu Lys Gly Asp Arg Gly Asp Pro Gly
145                 150                 155                 160
Glu Thr Gly Ala Ser Gly Glu Ser Gly Leu Ala
                165                 170

SEQ ID No.:3
SEQUENCE LENGTH: 3
SEQUENCE TYPE: amino acid
TOPOLOGY: linear
MOLECULAR TYPE: peptide

FEATURE
LOCATION:2
OTHER INFORMATION: 2nd amino acid is a protein-constituting amino acid.
LOCATION:3
OTHER INFORMATION: 3rd amino acid is a protein-constituting amino acid.
SEQUENCE:
Gly Xaa Xaa
1

SEQ ID No.:4
SEQUENCE LENGTH: 28
SEQUENCE TYPE: nucleic acid
STRADEDNESS: single
TOPOLOGY: linear
MOLECULAR TYPE: other nucleic acid, synthesized DNA
SEQUENCE:
GGCTCGAGGG GGAGAGTGGG CTTGCAGA 28

SEQ ID No.:5
SEQUENCE LENGTH: 28
SEQUENCE TYPE: nucleic acid
STRADEDNESS: single
TOPOLOGY: linear
MOLECULAR TYPE: other nucleic acid, synthesized DNA
SEQUENCE:
GGGAATTCTC AAAACTCGCA GATCACAA 28

## Claims

1. Recombinant conglutinin comprising a native conglutinin fragment, wherein said recombinant conglutinin comprises a collagen region having two units of amino acids sequence of Gly-Xaa-Xaa (SEQ ID No. 3), a neck region of the native conglutinin and a carbohydrate recognition domain of the native conglutinin, and 2nd and 3rd amino acids in said amino acid sequence of Gly-Xaa-Xaa are protein-constituting amino acid.

2. A method for producing the recombinant conglutinin comprising a native conglutinin fragment comprising the steps of:
(a) preparing a vector inserted thereinto cDNA corresponding to 613 bp through 1113 bp of the native conglutinin DNA,
(b) obtaining transformants by introducing said vector into Escherichia coli JM109,
(c) incubating said transformants in an apropriate medium,
(d) infecting said incubated transformants with phage, and
(e) collecting recombinant conglutinin from the phage-infected transformants,
wherein said recombinant conglutinin comprises a collagen region having two units of amino acids sequence of Gly-Xaa-Xaa (SEQ ID No. 3), a neck region of the native conglutinin and a carbohydrate recognition domain of the native conglutinin, and 2nd and 3rd amino acids in said amino acid sequence of Gly-Xaa-Xaa are protein-constituting amino acid.

3. A method for detecting an anti-virus activity of the collectins comprising the steps of:
(a) preparing cells infected with virus(es),
(b) co-presenting the infected cells with the collectins, and
(c) evaluating a inhibition level on budding of viruses in said cells.

4. The method for detecting the anti-virus activity according to the claim 3, wherein said collectins are materials selected from the group consisting of the mannan-binding protein (MBP), the human mannan-binding protein (hMBP), the conglutinin and the recombinant conglutinin.

5. The method for detecting the anti-virus activity according to the claim 3 or 4 wherein said virus is Influenza A virus.

6. Mannan-binding protein (MBP) having anti-Influenza A virus activity.

7. Human Mannan-binding protein (hMBP) having anti-Influenza A virus activity.
